# EUROPEAN PATENT SPECIFICATION

(11) **EP 2 594 551 B1**
(45) Date of publication and mention of the grant of the patent: **19.02.2025**
(21) Application number: 12192822.0
(22) Date of filing: 18.08.2006
(51) Int. Cl.: A61K 31/295, A61K 33/26, C07C 51/42, C07C 51/41, A61P 13/12, A61P 3/00, A61P 3/12, A61P 3/14, A61P 7/00, A61P 9/00, A61P 27/02, A61P 7/08

(54) **Uses of pharmaceutical-grade ferric organic compounds**
Verwendungen von eisenhaltigen organischen Verbindungen pharmazeutischer Qualität
Utilisations des composés organiques ferriques de qualité pharmaceutique

(30) Priority: 18.08.2005 US 206981; 19.08.2005 US 709511 P
(43) Date of publication of application: 22.05.2013
(62) Divisional of application: 06813544.1
(73) Proprietor: Panion & BF Biotech Inc., Taipei (TW)
(72) Inventor: Chan, Keith, Rockville, MD Maryland 20850 (US); Town, Winston, Hong Kong (CN)
(74) Representative: Cabinet Nony

(56) References cited:
- WO-A2-2004/074444
- US-A- 5 753 706
- US-A1- 2006 020 026
- US-B2- 6 903 235

## Description

### TECHNICAL FIELD

This invention relates to a use of pharmaceutical-grade ferric organic compounds, such as ferric citrate.

### BACKGROUND OF THE INVENTION

### Uses of Iron Compounds

Ferric iron containing compounds are useful in the treatment of a number of disorders, including, but not limited to, hyperphosphatemia and metabolic acidosis. See Hsu et al., "New Phosphate Binding Agents: Ferric Compounds", J Am Soc Nephrol, Vol. 10, Pages 1274-1280, 1999. Previous studies and inventions have reported the use of ferric compounds in binding with dietary phosphates, and such ferric compounds are potentially useful for the treatment of hyperphosphatemia in renal failure patients (U.S. Patent No. 5,753,706, 1998; U.S. Patent No. 6,903,235, 2005; CN 1315174, 2001; Yang W.C., et al., Nephrol. Dial. Transplant 17:265:270 (2002)). Elevated amounts of phosphate in the blood can be removed by administering compounds such as ferric citrate. Once in solution, the ferric iron binds phosphate, and the ferric phosphate compounds precipitate in the gastrointestinal tract, resulting in effective removal of dietary phosphate from the body. It is also believed that the absorbed citrate from ferric citrate is converted to bicarbonate which corrects metabolic acidosis, a condition common in renal failure patients. U.S Patent publication No. US5753706A relates to the control of phosphate retention in patients suffering from renal failure and associated hyperphosphatemia.

Int'l App. No. PCT/US2004/004646, filed February 18, 2004, published under Int'l Publication No. WO2004/074444 A2 on September 2, 2004, discloses a method of preparing ferric organic compounds, including ferric citrate that remains soluble over a wider range of pH than previously described preparations, and which have a large active surface area. However, commercially scalable manufacturing processes with quality control/analysis measures to ensure and/or to verify the compliance of the pharmaceutical-grade ferric citrate or ferric organic compounds with established standards or specifications were not previously disclosed.

Accordingly, there exists a need for a scalable process for synthesizing pharmaceutical-grade ferric organic compounds or ferric citrate for human use. The process needs to consistently produce ferric organic compounds or ferric citrate of the required pharmaceutical grade.

This invention discloses dosage for ferric citrate for treating of a human or non-human subject or patient.
in need of the compound. Different routes of administration are explored.

### SUMMARY OF THE INVENTION

The scope of the present invention is defined in the claims; any other disclosure in the present description or figures regardless of whether it is indicated as being preferred or exemplified, is provided for information purposes, only.

Any references in the description to methods of treatment refer to the compounds, pharmaceutical compositions and medicaments of the present invention for use in a method for treatment of the human (or animal) body by therapy.

In accordance with these and other objects of the invention, a brief summary of the present invention is presented. Detailed descriptions of a preferred exemplary embodiment adequate to allow those of ordinary skill in the art to make and use the invention concepts will follow in later sections.

Disclosed herein but not claimed is a manufacturing and quality control process/analysis for making a pharmaceutical-grade ferric citrate that consistently complies with the established Manufacture Release Specification. The process can be adapted to produce multi-kilogram batches of pharmaceutical-grade ferric citrate, and can be readily scaled up to provide additional manufacturing capacity for ferric citrate.

An illustrative embodiment of the manufacturing method may be exemplified by the following sequence of steps for preparing pharmaceutical-grade ferric citrate comprising the steps of: (a) dissolving an appropriate amount of Ferric chloride hexahydrate in water to form a Ferric chloride hexahydrate solution; (b) dissolving an appropriate amount of NaOH in water to form a NaOH solution; (c) mixing the Ferric chloride hexahydrate solution and NaOH solution to form a solution with Fe(OH)₃ precipitate; (d) maintaining the pH of the solution with Fe(OH)₃ precipitate above 7.0; (e) isolating the Fe(OH)₃ precipitate; (f) washing the Fe(OH)₃ precipitate three times with water; (g) suspending the Fe(OH)₃ precipitate in water; (h) adding citric acid to the Fe(OH)₃ precipitate to form a ferric-organic acid solution; (i) stirring and heating the ferric-organic acid solution at 90-100°C for 30 to 120 minutes; (j) removing solids in the ferric-organic acid solution by adding citric acid; (k) allowing the ferric-organic acid solution to cool to below 30°C; (1) maintaining the pH of the ferric-organic acid solution to between 0.8-1.5; (m) filtering the ferric-organic acid solution to obtain a liquid filtrate; (n) mixing acetone and liquid filtrate to form ferric citrate; (o) isolating ferric citrate; (p) washing ferric citrate with acetone three times; and (q) drying the ferric citrate.

A large-scale production scheme for pharmaceutical-grade ferric citrate comprising the steps of: (a) mixing an appropriate amount of NaOH and Ferric chloride hexahydrate in a suitable reactor to form a ferric hydroxide slurry with ferric hydroxide precipitate; (b) maintaining the pH of the ferric hydroxide slurry to above 7.0; (c) isolating the ferric hydroxide precipitate from the ferric hydroxide slurry using pressure filtration; (d) washing the ferric hydroxide precipitate three times; (e) maintaining the %Cl in the ferric hydroxide precipitate to below 5%; (f) isolating the washed ferric hydroxide precipitate using pressure filtration; (g) mixing citric acid with washed ferric hydroxide precipitate to form a ferric organic acid solution; (h) stirring and maintaining the temperature of the ferric organic acid solution at 80±5°C for 2 hours; (i) allowing the ferric organic acid solution to cool to 60°C; (j) maintaining the pH of the ferric organic acid solution to between 0.8 to 1.5 and the amount of Fe in the ferric organic acid solution to ≥ 85% of Fe added in step (a); (k) filtering the ferric organic acid solution using pressure filtration to obtain a liquid filtrate; (1) mixing the liquid filtrate with acetone to obtain ferric citrate; (m) isolating ferric citrate using pressure filtration; (n) washing the ferric citrate with acetone; (o) isolating the washed ferric citrate using pressure filtration; (p) drying the washed ferric citrate in fluidized bed dryer; and (q) maintaining the organic volatile impurities to ≤ 1000ppm acetone.

Also disclosed but not claimed are various intermediate compositions that may be useful in the preparation of the pharmaceutical-grade ferric citrate. The intermediate compositions encompassed herein include solids, liquids or multiphase mixtures. A liquid intermediate composition comprising the pharmaceutical-grade ferric citrate may be an aqueous composition or an organic solvent-based composition. A multiphase composition may encompass both aqueous and organic phases.

Methods of storing, packaging and using the various intermediate compositions are also disclosed herein but not claimed.

It is an object of the invention to provide and there is provided a pharmaceutical composition for use in treating hyperphosphatemia, the composition comprising a form of pharmaceutical-grade ferric citrate and a pharmaceutically suitable carrier, wherein the amount of pharmaceutical grade ferric citrate administered per day is 6 grams per day, and wherein the pharmaceutical grade ferric citrate is administered in a tablet or capsule form, wherein the pharmaceutical grade ferric citrate is obtainable by a method comprising the steps of:
(a) dissolving an appropriate amount of ferric chloride hexahydrate in water to form a ferric chloride hexahydrate solution;
(b) dissolving an appropriate amount of NaOH in water to form a NaOH solution;
(c) mixing the ferric chloride hexahydrate solution and NaOH solution to form a solution with Fe(OH)₃ precipitate;
(d) maintaining the pH of the solution with Fe(OH)3 precipitate above 7.0;
(e) isolating the Fe(OH)₃ precipitate;
(f) washing the Fe(OH)₃ precipitate three times with water;
(g) suspending the Fe(OH)₃ precipitate in water;
(h) adding citric acid to the Fe(OH)₃ precipitate to form a ferric-organic acid solution;
(i) stirring and heating the ferric-organic acid solution at 90-100°C for 30 to 120 minutes;
(j) removing solids in the ferric-organic acid solution by adding citric acid;
(k) allowing the ferric-organic acid solution to cool to below 30°C;
(1) maintaining the pH of the ferric-organic acid solution to between 0.8-1.5;
(m) filtering the ferric-organic acid solution to obtain a liquid filtrate;
(n) mixing acetone and liquid filtrate to form ferric citrate;
(o) isolating ferric citrate;
(p) washing ferric citrate with acetone three times; and
(q) drying the ferric citrate.

Aptly, the pharmaceutical composition is in an orally administrable form.

Aptly, the orally administrable form is a tablet or capsule.

The pharmaceutically useful composition further comprises any pharmaceutically acceptable carrier, adjuvant, filler or delivery vehicle suitable for administering to a subject or human patient, an effective amount of the pharmaceutical-grade ferric citrate.

Further embodiments of the pharmaceutical compositions include solids, liquids, or semi-solid forms, such as gels, syrups, chewables or pastes.

An example of a method for using the pharmaceutical compositions encompasses treating disorders resulting from elevated blood levels of phosphates, i.e., hyperphosphateimia, in a subject or a human patient. Such disorders are exemplified by renal failure or the progression of renal failure, mineralization of soft tissues, hyperparathyroidism as well as other complications.

Also disclosed is a pharmaceutical composition comprising:
(a) an amount of a pharmaceutical-grade ferric organic compound effective to achieve a decrease in the serum phosphate level of a subject or patient;
(b) a pharmaceutically suitable carrier; and
wherein the ferric organic compound complies with at least one of the limits in the manufacture release specification in Table A.

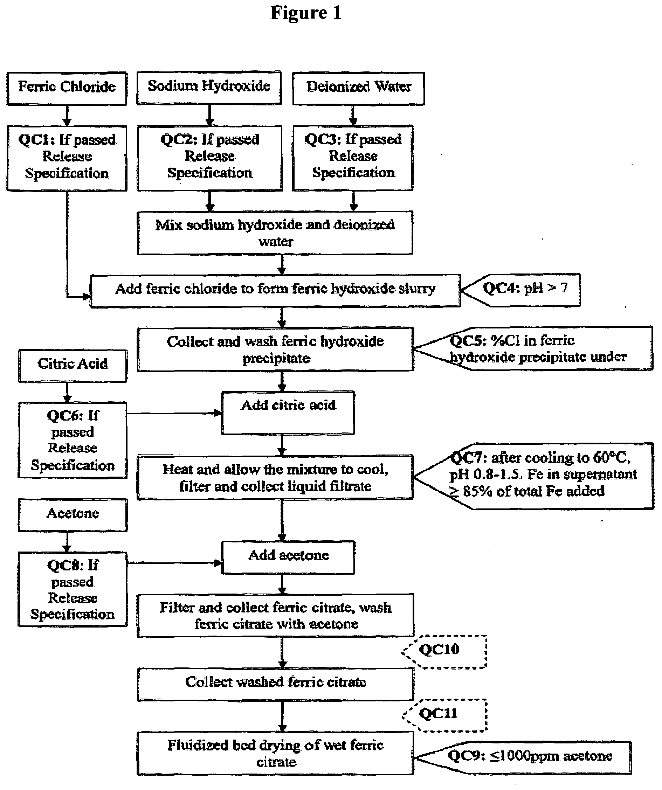

The pharmaceutical composition described herein may be prepared by a method shown in Figure 1, 2, 3 or 4. Compositions may be prepared with materials satisfying at least one limit disclosed in each of the release specifications of either of the relevant Tables B to F.

An additional embodiment encompasses compositions of pharmaceutical-grade ferric citrate, prepared according to methods comprising the steps of:
(a) dissolving an appropriate amount of ferric chloride hexahydrate in water to form a ferric chloride hexahydrate solution;
(b) dissolving an appropriate amount of NaOH in water to form a NaOH solution;
(c) mixing the ferric chloride hexahydrate solution and NaOH solution to form a solution with Fe(OH)3 precipitate;
(d) maintaining the pH of the solution with Fe(OH)₃ precipitate above 7.0;
(e) isolating the Fe(OH)₃ precipitate;
(f) washing the Fe(OH)₃ precipitate three times with water;
(g) suspending the Fe(OH)₃ precipitate in water;
(h) adding citric acid to the Fe(OH)₃ precipitate to form a ferric-organic acid solution;
(i) stirring and heating the ferric-organic acid solution at 90-100°C for 30 to 120 minutes;
(j) removing solids in the ferric-organic acid solution by adding citric acid;
(k) allowing the ferric-organic acid solution to cool to below 30°C;
(l) maintaining the pH of the ferric-organic acid solution to between 0.8-1.5;
(m) filtering the ferric-organic acid solution to obtain a liquid filtrate;
(n) mixing acetone and liquid filtrate to form ferric citrate;
(o) isolating ferric citrate;
(p) washing ferric citrate with acetone three times;
(q) drying the ferric citrate; and
(r) performing quality control tests as shown in Figures 1, 2 or 3.

It is advantageous to scale-up the method of preparation. Thus, an industrial-scale method is embodied by a method for large-scale production of pharmaceutical-grade ferric citrate comprising the steps of:
(a) mixing an appropriate amount of NaOH and Ferric chloride hexahydrate in a suitable reactor to form a ferric hydroxide slurry with ferric hydroxide precipitate;
(b) maintaining the pH of the ferric hydroxide slurry to above 7.0;
(c) isolating the ferric hydroxide precipitate from the ferric hydroxide slurry using pressure filtration;
(d) washing the ferric hydroxide precipitate three times;
(e) maintaining the %Cl in the ferric hydroxide precipitate to below 5%;
(f) isolating the washed ferric hydroxide precipitate using pressure filtration;
(g) mixing citric acid with washed ferric hydroxide precipitate to form a ferric organic acid solution;
(h) stirring and maintaining the temperature of the ferric organic acid solution at 80±5°C for 2 hours;
(i) allowing the ferric organic acid solution to cool to 60°C;
(j) maintaining the pH of the ferric organic acid solution to between 0.8 to 1.5 and the amount of Fe in the ferric organic acid solution to ≥ 85% of Fe added in step (a);
(k) filtering the ferric organic acid solution using pressure filtration to obtain a liquid filtrate;
(1) mixing the liquid filtrate with acetone to obtain ferric citrate;
(m) isolating ferric citrate using pressure filtration;
(n) washing the ferric citrate with acetone;
(o) isolating the washed ferric citrate using pressure filtration;
(p) drying the washed ferric citrate in fluidized bed dryer; and
(q) maintaining the organic volatile impurities to ≤ 1000ppm acetone;
and wherein the ferric citrate complies with at least one of the limits in the manufacture release specification in Table A.

The compositions encompassing pharmaceutical grade ferric citrate are suitable for treating hyperphosphatemia, or other disorders characterized by high serum phosphate levels. Therefore, the invention encompasses treating subjects or patients with various renal diseases resulting from hyperphosphatemia; e.g., End Stage Renal Diseases (ESRD), Chronic Kidney Disease (CKD) or other relate kidney diseases, or subjects and patients who are on dialysis but not limited to hemodialysis.

An embodiment of a method for using the pharmaceutical composition encompasses treating a human or non-human subject or patient with chronic kidney disease. There are generally five clinical stages of chronic kidney disease, numbered 1 to 5, wherein stage 1 is the least severe and stage 5 the most severe. In the early stages, e.g., stages 1 and 2, dialysis is not required. As chronic kidney disease progresses to stage 5, a patient may require dialysis treatment three times per week. It should be noted that elevated phosphate levels are observed at all stages of chronic kidney disease. A method of treating a subject or person with early or mid-stage chronic kidney disease comprises treating with a composition comprising pharmaceutical-grade ferric citrate in order to achieve a lower serum phosphate level.

It is a further embodiment of the invention to provide a method of treating a human or non-human subject or patient with late-stage chronic kidney disease who undergo hemodialysis, by administering a composition comprising pharmaceutical-grade ferric citrate. It is known that hemodialysis is not sufficiently effective in reducing serum phosphate level. The treatment of a subject or person with late stage kidney disease is applicable whether or not the subject or person is currently undergoing hemodialysis treatment.

An additional embodiment of the invention is a method of treating a subject or person with chronic kidney disease and undergoing peritoneal dialysis with the pharmaceutical-grade ferric citrate-containing compositions described. It is known that peritoneal dialysis is not sufficiently effective in reducing serum phosphate levels.

An additional embodiment is a method for using the pharmaceutical composition to inhibit or even reverse soft tissue mineralization, specifically calcification. Hyperphosphatemia may lead to increased calcium phosphate deposition in hard and soft tissues by increasing the likelihood of binding with free calcium to form insoluble calcium phosphate. Therefore, an effective dose of a composition comprising pharmaceutical-grade ferric citrate may decrease serum phosphate levels and result in a corresponding decrease in calcium phosphate deposition.

It is noteworthy that whereas ferric ion forms insoluble precipitates with phosphate-containing compounds in the lumen of the gastroinstestinal tract, the citrate component is absorbed and functions as a calcium chelator. Because chelated calcium is not available for calcium phosphate formation, administering pharmaceutical-grade ferric citrate decreases may lead to reductions in both serum calcium and phosphate. This may also be expressed as leading to a decrease in the serum calcium-phosphate product. Reducing serum calcium and phosphate would be expected to reduce calcium phosphate deposition. The end result is reversing, i.e., solubilizing or dissolving, the deposited calcium phosphate.

The decalcifying of a calcified soft tissue, e.g., the sclera of the eye, may be achieved by administering pharmaceutical-grade ferric citrate. It is known among persons of ordinary skill in the relevant medical arts that patients with kidney disease receiving doses of ferric citrate have shown reversal of calcium deposits on the eye. Therefore, an embodiment of the invention is directed to the decalcification of soft tissue such as the eye.

Kidney stones comprise calcium salts of oxalic acid or phosphates and are formed by mechanisms similar to those described above. Thus, pharmaceutical-grade ferric citrate, in another embodiment of the invention provides a method of treating kidney stones, i.e., renal calculi, by promoting their dissolution.

The compositions encompassing pharmaceutical grade ferric citrate may operate according to more than one mechanism. A plausible non-limiting mechanism of action may result from the ferric ion binding phosphate in the GI tract, thus forming an insoluble ferric phosphate precipitate. This, in turn, may result in decrease the uptake of phosphate and phosphate-containing compounds from the GI tract.

In view of such a mechanism, the administering of compositions encompassing pharmaceutical grade ferric citrate via an oral route is encompassed by the inventive methods described.

This invention provides a method of reversing, preventing or stabilizing soft tissue calcification of a subject, comprising administering to said subject and effective amount of a ferric citrate compound.

This invention further provides a method of reversing, preventing or stabilizing soft tissue calcification of a subject, comprising administering to said subject and effective amount of a ferric citrate compound, wherein the ferric citrate compound is prepared according to a method as shown in Figures 1-4.

This invention provides a method of reversing, preventing or stabilizing soft tissue calcification of a subject, comprising administering to said subject and effective amount of a ferric citrate compound, wherein the ferric citrate compound is prepared according a method comprising the steps of:
The compositions encompassing pharmaceutical grade ferric citrate may be administered for varying periods of time. In some embodiments, the tolerability of the compositions encompassing pharmaceutical grade ferric citrate allows for long term administration when necessary.

### DETAILED DESCRIPTION OF THE FIGURES

In drawings which illustrate specific embodiments of the invention:
**Figure 1** is a schematic diagram outlining the general method for synthesis of pharmaceutical-grade ferric citrate including in-process quality control measures to ensure the final ferric citrate product complies with the established Manufacture Release Specification as shown in Table A.
**Figure 2** is a schematic diagram outlining a method of making pharmaceutical-grade ferric citrate.
**Figure 3** shows an overview of scalable pharmaceutical-grade ferric citrate manufacturing and quality control process.
**Figure 4** is a schematic diagram of scalable pharmaceutical-grade ferric citrate manufacturing and quality control process.

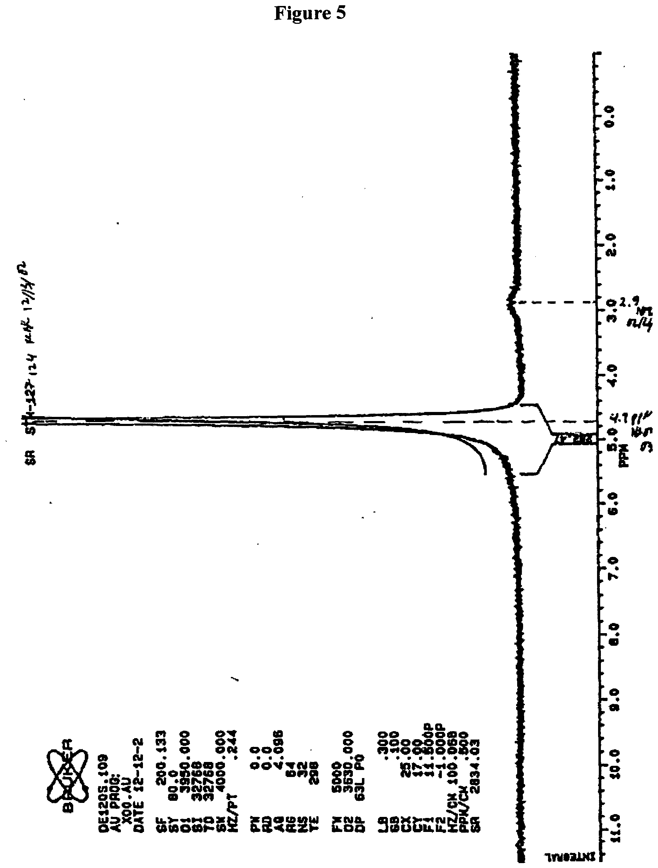

**Figure 5** shows the representative proton NMR spectrum for a pharmaceutical-grade ferric citrate according to the invention.
**Figure 6** shows the representative FTIR spectrum for pharmaceutical-grade ferric citrate according to the invention.
**Figure 7** is a comparison of intrinsic dissolution rates at pH 1.0 and 8.0 for pharmaceutical grade and chemical grade ferric citrate.
**Figure 8** is a schematic diagram outlining the general method for synthesis of pharmaceutical-grade ferric organic compound including in-process quality control measures to ensure the final ferric organic compound complies with established Manufacture Release Specification.
**Figure 9** **is a** summary of clinical results for pharmaceutical-grade ferric citrate.
**Figure 10** is a summary of the efficacy data for pharmaceutical-grade ferric citrate.
**Figure 11** is a summary of the safety data from a clinical study.
**Figure 12** is a comparison of the safety profiles of chemical grade and pharmaceutical grade ferric citrates.
**Figure 13** is a comparison of the efficacy profiles of chemical grade and pharmaceutical grade ferric citrates.

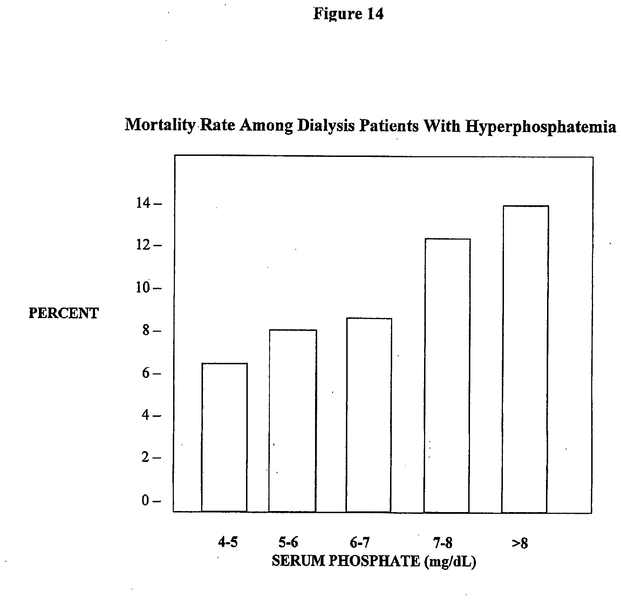

**Figure 14** shows a bar graph of the relationship between the rate of dialysis patient mortality and hyperphosphatemia.

### DETAILED DESCRIPTION OF THE INVENTION

In drawings specific embodiments of the invention are illustrated. Accordingly, the specification and drawings are to be regarded in an illustrative, rather than a restrictive, sense.

Throughout this application, references are made to the United Stats Pharmacopeia (USP), and the latest edition of the USP, USP 28.

A method of preparing pharmaceutical-grade ferric citrate, comprises the steps of: (a) dissolving an appropriate amount of Ferric chloride hexahydrate in water to form a Ferric chloride hexahydrate solution; (b) dissolving an appropriate amount of NaOH in water to form a NaOH solution; (c) mixing the Ferric chloride hexahydrate solution and NaOH solution to form a solution with Fe(OH)₃ precipitate; (d) maintaining the pH of the solution with Fe(OH)₃ precipitate above 7.0; (e) isolating the Fe(OH)₃ precipitate; (f) washing the Fe(OH)₃ precipitate three times with water; (g) suspending the Fe(OH)₃ precipitate in water; (h) adding citric acid to the Fe(OH)₃ precipitate to form a ferric-organic acid solution; (i) stirring and heating the ferric-organic acid solution at 90-100°C for 30 to 120 minutes; (j) removing solids in the ferric-organic acid solution by adding citric acid; (k) allowing the ferric-organic acid solution to cool to below 30°C; (1) maintaining the pH of the ferric-organic acid solution to between 0.8-1.5; (m) filtering the ferric-organic acid solution to obtain a liquid filtrate; (n) mixing acetone and liquid filtrate to form ferric citrate; (o) isolating ferric citrate; (p) washing ferric citrate with acetone three times; and (q) drying the ferric citrate.

A method for scalable or large-scale production of pharmaceutical-grade ferric citrate comprises the steps of: (a) mixing an appropriate amount of NaOH and Ferric chloride hexahydrate in a suitable reactor to form a ferric hydroxide slurry with ferric hydroxide precipitate; (b) maintaining the pH of the ferric hydroxide slurry to above 7.0; (c) isolating the ferric hydroxide precipitate from the ferric hydroxide slurry using pressure filtration; (d) washing the ferric hydroxide precipitate three times; (e) maintaining the %Cl in the ferric hydroxide precipitate to below 5%; (f) isolating the washed ferric hydroxide precipitate using pressure filtration; (g) mixing citric acid with washed ferric hydroxide precipitate to form a ferric organic acid solution; (h) stirring and maintaining the temperature of the ferric organic acid solution at 80±5°C for 2 hours; (i) allowing the ferric organic acid solution to cool to 60°C; (j) maintaining the pH of the ferric organic acid solution to between 0.8 to 1.5 and the amount of Fe in the ferric organic acid solution to ≥ 85% of Fe added in step (a); (k) filtering the ferric organic acid solution using pressure filtration to obtain a liquid filtrate; (1) mixing the liquid filtrate with acetone to obtain ferric citrate; (m) isolating ferric citrate using pressure filtration; (n) washing the ferric citrate with acetone; (o) isolating the washed ferric citrate using pressure filtration; (p) drying the washed ferric citrate in fluidized bed dryer; and (q) maintaining the organic volatile impurities to ≤ 1000ppm acetone.

The ferric chloride hexahydrate may comply with the release specification as shown in Table B; the citric acid may comply with the release specification as shown in Table F; the water may comply with the release specification as shown in Table D; the acetone may comply with the release specification as shown in Table E; and the sodium hydroxide may comply with the release specification as shown in Table C.

The ferric citrate may be dried using a fluidized bed dryer or is dried under vacuum.

The process as described above may further comprise testing the ferric citrate for compliance with the release specification as shown in Table A. Testing may comprise performing at least one test selected from the group consisting of: assay content purity of ferric citrate and ferric citrate monohydrate; assay content of citric acid; assay content of detectable ferric citrate related substances; assay content of ferric ion; elemental iron impurity test; limit of ferrous iron test; loss on drying test; hydrate test (water content by differential scanning calorimetry); hydrate test (water content by karl Fischer Titration); trace or heavy metals test (As, Ca, Cd, Cu, Fe, Hg, Na, Pb, Sr, Zn); limit of oxalic acid test; identification A for ferric salts test; identification B by FTIR test; insoluble substances test; limit of ammonium test; limit of chloride test; limit of nitrate test; limit of tartrate test; residue on ignition test; organic volatile impurities test; and microbial, mold and yeast test.

This invention provides a composition comprising the ferric citrate as described above and in the claims for treating hyperphosphatemia.

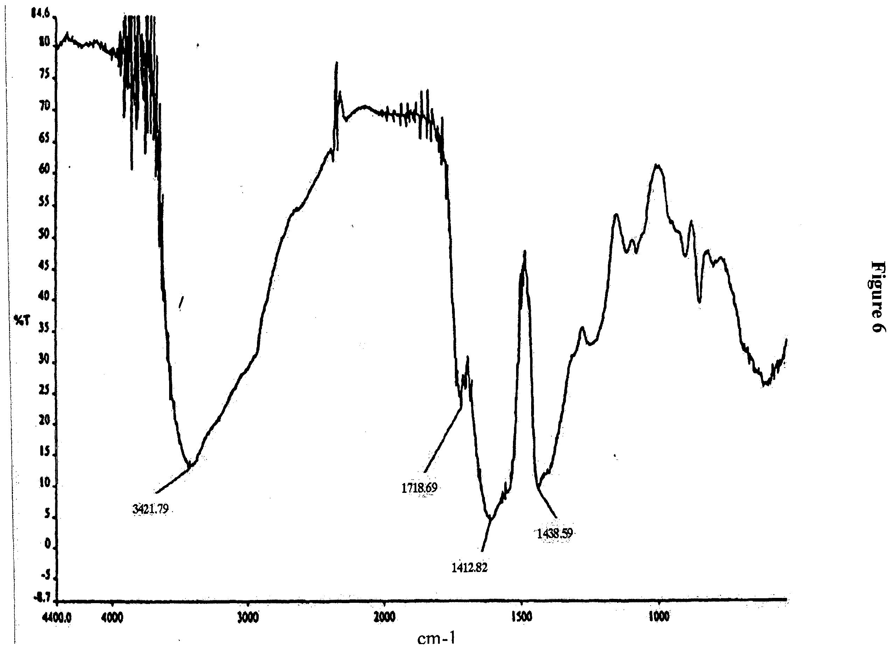

This invention provides a pharmaceutical-grade ferric citrate prepared according to the methods described above, wherein the ferric citrate produces the peak as shown in Figure 6 when subjected to NMR spectroscopy analysis.

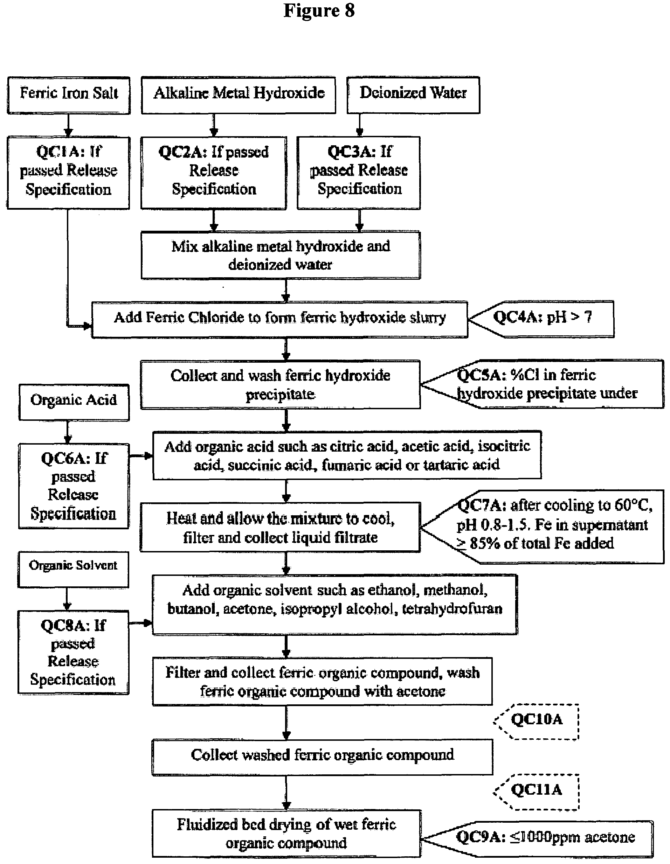

This invention provides a pharmaceutical-grade ferric citrate prepared according to the methods described above, wherein the ferric citrate possesses the dissolution rates as shown in Figure 8.

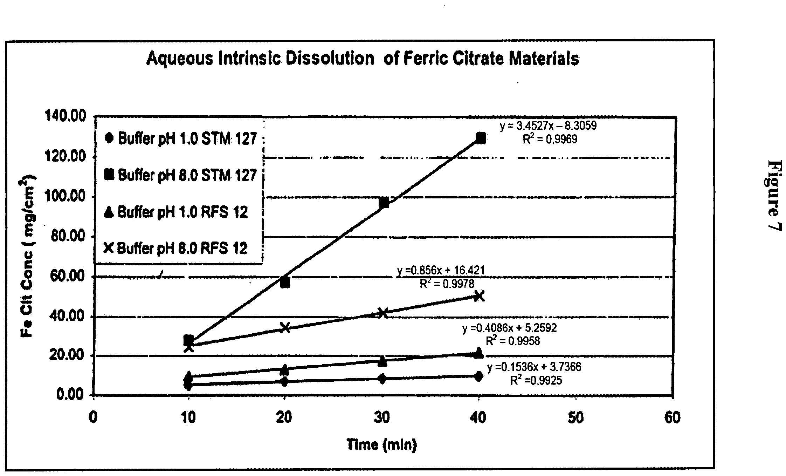

This invention provides a pharmaceutical-grade ferric citrate prepared according to the methods described above, wherein the ferric citrate produces the spectral data as shown in Figure 7 when subjected to Fourier Transform Infrared Spectrometry (FTIR) spectrum analysis.

This invention provides a composition comprising the ferric citrate as described above for treating hyperphosphatemia.

The pharmaceutical-grade ferric citrate according to the invention is useful for treating a subject suffering from hyperphosphatemia. In an embodiment, the subject is a human being.

The invention also provide a use for a composition comprising pharmaceutical-grade ferric citrate effective for one or more of the following uses,
(a) treating hyperphosphatemia and the following uses (b) to (e), however, only in as far as resulting from hyperphosphatemia:
(b) decreasing mortality rate in dialysis patients;
(c) inhibiting calcium phosphate deposition;
(d) decreasing serum calcium-phosphate product ([Ca] x [P]); and
(e) decreasing serum calcium levels.

The use may further encompass at least partly relying on reducing serum levels of calcium and phosphate ions.

The uses as stated above may apply to soft tissue such as a blood vessel or an eye.

The uses as stated may be carried out by administering over a long-term.

The invention allows for the above-stated uses to be achieved wherein the use is accompanied by less gastrointestinal adverse side effects than if the composition comprised chemical grade ferric citrate.

In accordance with the above-stated uses, the invention provides for a method for treating a disorder characterized by a high serum phosphate level comprising administering an effective amount of a composition comprising pharmaceutical-grade ferric citrate.

The invention also provides for the use wherein an effective amount of pharmaceutical-grade ferric citrate is administered in the form of a tablet or a capsule.

The invention also provides for the use wherein the effective amount of pharmaceutical-grade ferric citrate is a daily effective amount of 6 grams.

In general, hyperphosphatemia is prevalent in patients with chronic renal failure and in patients on dialysis. There is also evidence that indicate elevated serum phosphorus, calcium-phosphorus product (CaxP) and parathyroid hormone (PTH) levels contribute to increased incidence of vascular, visceral, peripheral vascular and soft tissue calcification in renal disease patients.

It may be possible to prevent or reverse calcification in renal disease patient or in a normal person. For example, dissolving kidney stones that may accompany renal failure.

Phosphorous exerts a negative impact on vascular calcification by direct participation in the CaxP and indirectly in the pathogenesis and progression of hyperthyroidism. Serum calcium and phosphorous are metastable under normal circumstances, which means that their concentrations are not sufficient to produce spontaneous precipitation. However, once the calcification process begins, the concentrations are sufficient to support crystal proliferation.

Available evidence confirms a high prevalence of underlying vascular disease and structural heart disease in patients with severe chronic renal failure. These structural lesions are then exposed to elevated serum phosphorus, CaxP, and PTH(1).

Factors which are considered likely to contribute to elevated serum phosphorus and CaxP include administration of calcium-containing phosphorus binders. The calcium-containing phosphorus binders, such as calcium acetate are prescribed to many new hemodialysis (HD) and peritoneal dialysis (PD) patients, thus providing a large source of exogenous calcium to the GI tract.

Calcification also extends beyond renal disease patients and can include anyone who is over the age of 40. While the leading cause of death in the United States is acute myocardial infarction and stroke, hypercholesteromia contribute to only 15% of the deaths in this category and 85% is caused by ventricular calcification.

It has been shown that abnormalities in serum phosphorous, CaxP and PTH levels can result in vascular, visceral and/or soft tissue calcification. For example, calcifications of myocardium, coronary arteries, cornea can lead to the development of a number of clinically significant complications including myocardial ischemia, myocardial infarction, impaired myocardial function, congestive heart failure, cardiac valve insufficiency and blindness.

Accordingly, there exists a need for methods of managing or reducing serum phosphorous as a means of treating numerous medical disorders. The method includes administering a phosphate binder which does not adversely affect serum calcium levels and does not cause toxic side effects in the patient.

### EXAMPLES

In examples which are intended to illustrate embodiments of the invention:

### Example 1. General Method for Synthesis of a Pharmaceutical-Grade Ferric Citrate

Referring to Figure 1, which shows a general method of synthesis of the pharmaceutical-grade ferric citrate according to the invention, the flow chart shows implementation of quality control measures at selected stages of the synthesis process to ensure the final ferric citrate product complies with the Manufacture Release Specification as shown in Table A or any established Manufacture Release Specification for pharmaceutical-grade ferric citrate which have been approved or are suitable for human use. Other quality control measures or procedures, which are readily apparent to one of ordinary skill in the art, can be used or incorporated into the pharmaceutical-grade synthesis process to maintain the quality and purity of the final product and to increase the efficiency and yield of the synthesis process. See, for example, **QC10** and **QC11** in Figure 1.

Examples of quality control measures employed in the synthesis process include: **(QC4)** maintaining pH of the ferric hydroxide slurry above 7.0; **(QC5)** maintaining the %Cl in ferric hydroxide precipitate below 5%; **(QC7)** maintaining the pH of the mixture between 0.8-1.5 and the Fe in mixture ≥ 85% of total Fe added after adding citric acid to ferric hydroxide precipitate; and **(QC9)** maintaining the level of acetone to ≤1000ppm during the drying stage.

The raw materials, i.e., ferric chloride, deionized water, citric acid, acetone, sodium hydroxide, may pass release specifications, such as those provided on Table B-F, before being used in the synthesis process. See Figure 1, **QC1-QC3, QC6** and **QC8.**

### Example 1A. General Method for Synthesis of a Pharmaceutical-Grade Ferric Organic Compounds

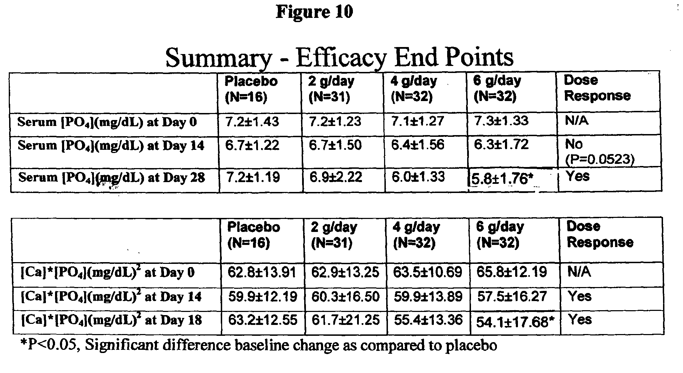

Referring to Figure 10, which shows a general method of synthesis of the pharmaceutical-grade ferric organic compounds according to the invention, the flow chart shows implementation of quality control measures at selected stages of the synthesis process to ensure the final ferric citrate product complies with established Manufacture Release Specification for pharmaceutical-grade ferric organic compounds which have been approved or are suitable for human use. Other quality control measures or procedures, which are readily apparent to one of ordinary skill in the art, can be used or incorporated into the pharmaceutical-grade synthesis process to maintain the quality and purity of the final product and to increase the efficiency and yield of the synthesis process. See, for example, **QC10A** and **QC11A** in Figure 10.

Examples of quality control measures employed in the synthesis process include: **(QC4A)** maintaining pH of the ferric hydroxide slurry above 7.0; **(QC5A)** maintaining the %Cl in ferric hydroxide precipitate below 5%; **(QC7A)** maintaining the pH of the mixture between 0.8-1.5 and the Fe in mixture ≥ 85% of total Fe added after adding organic acid to ferric hydroxide precipitate; and **(QC9A)** maintaining the level of organic solvent to ≤1000ppm during the drying stage.

The raw materials, i.e., ferric iron salt, deionized water, organic acid, organic solvent, alkaline metal hydroxide, may pass release specifications before being used in the synthesis process. See Figure 1, **QC1A-QC3A, QC6A** and **QC8A.** The organic acid can comprise citric acid, acetic acid, isocitric acid, succinic acid, fumaric acid, tartaric acid, or any other suitable organic acid. The organic solvent can comprise ethanol, methanol, butanol, acetone, isopropyl alcohol, tetrahydrofuran, or any other suitable organic solvent.

### Example 2. Solubility Profile of Ferric Organic Compounds According to the Invention

The ferric organic compounds produced according to the methods described above are more soluble than commercially available ferric organic compounds, over a wider range of pH levels. This increase in solubility of the ferric organic compounds of the present invention is believed to be a result of the unique significantly large active surface area of the ferric organic compounds of the present invention. For example, at pH 8.0, the intrinsic dissolution rate of ferric citrate of the present invention is 3.08 times greater than the commercially available ferric citrate. See Table 1.

The intrinsic dissolution rates of commercially available ferric citrate were compared with the ferric citrate of the present invention. The intrinsic dissolution rate is defined as the dissolution rate of pure substances under the condition of constant surface area. The dissolution rate and bioavailability of a drug substance is influence by its solid state properties: crystallinity, amorphism, polymorphism, hydration, solvation, particle size and particle surface area. The measured intrinsic dissolution rate is dependent on these solid-state properties and is typically determined by exposing a constant surface area of a material to an appropriate dissolution medium while maintaining constant temperature, stirring rate, and pH. The intrinsic dissolution rates are presented in Table 1.

**Table 1. Intrinsic dissolution rates of ferric citrate at 37 °C in solutions of pH 8**

| **Sample** | **Rate of Acetone Addition (ml/min)** | **Intrinsic Dissolution Rates (mg/cm2/min)** | **Mean Intrinsic Dissolution Rates (mg/cm2/min)** |
|---|---|---|---|
| RFS-12 (sigma / commercially available) | 10.0 | 0.83 | 0.83 |
| STM-134 (reference material) | 10.0 | 1.88 | 3.08 |
| PAN031203A (experimental batch 1) | 10.0 | 3.82 | |
| PAN031203B (experimental batch 2) | 10.0 | 4.00 | |
| PAN031203C (experimental batch 3) | 9.5 | 2.68 | |
| PAN031203D (experimental batch 4) | 40 | 2.95 | |
| PAN031203E (experimental batch 5) | 4.4 | 3.13 | |

For example, the BET active surface area of the ferric citrate of the present invention is at least 16 times larger than the commercially available ferric citrate. See Table 2.

The analysis of active surface area is based on BET theory which describes the phenomenon of mass and energy interaction and phase changes during gas adsorption onto solid surfaces and in pore spaces. In BET active surface area measurement, the volume of a monolayer of gas is determined which allows the surface area of the sample to be determined using the area occupied by a single layer of adsorbed gas molecule. Table 2 is a comparison of the active surface area of the ferric citrate of the present invention compared to the active surface area of commercially available ferric citrate compounds.

**Table 2. BET active surface areas of various forms of ferric citrate**

| **Sample** | **Mean Dissolution Rates (mg/cm2/min)** | **BET Active Surface Area** |
|---|---|---|
| RFS-12-1 (sigma / commercially available) | 0.76 | 0.61 |
| RFS-12-2 (sigma / commercially available) | | |
| STM-134-1 (reference material 1) | 2.47 | 16.17 |
| STM-134-2 (reference material 2) | | |
| STM-182-1 (lab-scale 500 g batch 1) | 2.61 | 19.85 |
| STM-182-2 (lab-scale 500 g batch 2) | | |

### Example 3. Use of Ferric Organic Compounds According to the Invention in the Treatment of Disorders

The ferric organic compounds produced according to the methods described above are useful in the treatment of hyperphosphatemia, metabolic acidosis, and any other disorders responsive to ferric organic compound therapy. Because the ferric organic compounds of the present invention are more soluble than commercially available ferric organic compounds, smaller amounts of the ferric organic compounds of the present invention can be used to effectively treat patients suffering from such disorders.

Improved aqueous solubility is particularly relevant to the use of the ferric organic compounds of the present invention in the treatment of disorders responsive to ferric organic compound therapy. Because the ferric organic compounds of the present invention are more soluble, they will be more effective when taken orally, and therefore can be taken in lower doses. The ferric organic compounds of the present invention are more soluble over a wider pH range than commercially available ferric organic compounds; therefore, the ferric organic compounds of the present invention can be more effective by being soluble in the small intestine.

For example, in an experiment simulating the alkaline condition in the small intestine, the ferric citrate of the present invention showed better dissolution rate than the commercially available ferric citrate. It is suggested that the ferric citrate of the present invention can be more effective by being more soluble in the small intestine. See Table 1. As a result, patients can take lower doses of medication with lower incidences of side effects.

In one embodiment of the invention, the ferric citrate of the present invention has a significantly higher rate of aqueous solubility under physiological conditions than commercially available forms of ferric citrate, and therefore the ferric citrate of the present invention is believed to provide a significant improvement in the orally effective use of ferric citrate at a reduced dosage. By reducing the orally effective dose of ferric citrate, it is believed that the ferric citrate of the present invention will provide a lower incidence of ulcerative gastrointestinal adverse effects associated with commercially available ferric citrate compounds. In addition, it is believed that the increased rate of dissolution of the ferric citrate of the present invention will provide a more rapid onset of action in binding to dietary phosphate.

The ferric organic compounds of the present invention can be administered in a number of forms, including orally administrable forms, which can comprise the ferric organic compounds of the present invention alone or in combination with a pharmaceutically acceptable carrier. The orally administrable form includes a tablet or a capsule. The composition can be administered to human beings or other animals suffering from illnesses responsive to ferric organic compound therapy.

### Example 4. A Method of Making a Pharmaceutical-grade Ferric Citrate

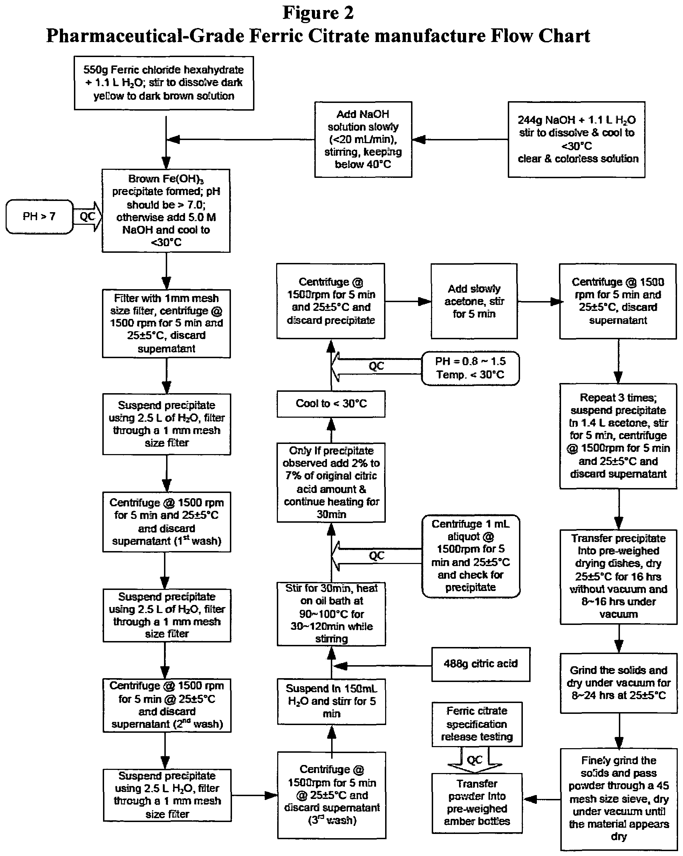

Described herein is a process for manufacturing pharmaceutical-grade ferric citrate suitable as an active pharmaceutical ingredient for human use. An overview of the Ferric Citrate Manufacture Flow Chart is shown in Figure 1. For a specific example, pharmaceutical-grade ferric citrate was produced using the procedure described below. Also see Figure 2.

### 4.1. Preparation of Ferric Chloride Solution

- Weigh 550 g of ferric chloride hexahydrate (correct for CoA purity) into a 1 L beaker.
- Transfer the ferric chloride hexahydrate into a 4 L Erlenmeyer flask.
- Measure 1.1 L of deionized water using a graduated cylinder. Use a small portion of the deionized water to rinse the beaker and transfer the water into the 4 L Erlenmeyer flask. Transfer the remaining water into the Erlenmeyer flask.
- Stir solution using a magnetic stirring bar until completely dissolved. The solution is a dark yellow to dark brown color.

### 4.2. Preparation of Sodium Hydroxide Solution

- Weigh 244 g of sodium hydroxide (correct for CoA purity) into a 500 mL beaker.
- Transfer the sodium hydroxide into a 2 L Erlenmeyer flask.
- Measure 1.1 L of deionized water using a graduated cylinder. Use a small portion of the deionized water to rinse the beaker and transfer the water into a 4 L Erlenmeyer flask. Transfer the remaining water into the Erlenmeyer flask slowly.
- In a fumehood, stir the solution using a stirring bar while adding the water and stir until completely dissolved. The solution is clear and colorless.
- Cool solution to below 30°C using a water bath.

### 4.3. Preparation of Ferric Hydroxide Intermediate

- Place a magnetic stirring bar into the ferric chloride solution and place the flask in a water bath. Set up on a stirring plate and start the stirring plate at a low speed.
- Add slowly the sodium hydroxide solution to the ferric chloride solution (at a rate of less than 20 mL/min) using an addition funnel and control the temperature of the reaction mixture below 40°C using the water bath and the rate of addition of sodium hydroxide.
- Continue to cool the brown viscous mixture to below 30°C using the water bath.
- The final pH should be above 7. Use a suitable volume of 5 M aqueous sodium hydroxide solution to correct the pH if not above 7. Measure and record the final pH. A dark brown precipitate of ferric hydroxide is formed.
- If required, cool the brown viscous mixture to below 30°C using the cold water bath and filter the ferric hydroxide suspension through 1 mm size stainless steel filter to break up large precipitates.
- Transfer equal amounts of ferric hydroxide suspension into four 500 mL centrifuge containers. Balance the weight of each centrifuge container using a top-loading balance before centrifugation.
- Centrifuge the ferric hydroxide suspension at 1500 rpm and 25±5°C for 5 minutes. Discard the supernatant.
- Measure 2.5 L of deionized water using a graduated cylinder and use approximately 1 L of water to re-suspend the ferric hydroxide precipitate from the centrifuge containers.
- Transfer the ferric hydroxide suspension into a 4 L Erlenmeyer flask fitted with a 1 mm size stainless steel filter over a glass funnel. Use the remaining 1.5 L of deionized water to rinse the containers and wash the precipitate retained on the stainless steel filter.
- Wash the precipitate two more times by repeating the steps beginning with "Transfer equal amounts..."
- After the third wash, recover the precipitate by repeating the steps beginning with "Transfer equal amounts..." and ending with "Centrifuge the ferric hydroxide suspension..."
- Re-suspend the precipitate in 150 mL of deionized water.

### 4.4. Preparation of Ferric Citrate

- Homogenize the ferric hydroxide precipitate using a mechanical stirrer for 5 min in a 2 L Erlenmeyer flask.
- Weigh 490 g of citric acid (correct for CoA purity) into a 500 mL beaker.
- Place a stir bar in the 2 L Erlenmeyer flask in an oil bath and stir at high speed.
- Add the citric acid into the ferric hydroxide suspension.
- Stir the solution for 30 minutes.
- Heat the mixture at 90 to 100°C (in oil bath) until the color changes from orange-brown to a clear black-brown (for 30 to 120 min) or until ferric hydroxide precipitate is dissolved.
- Take 1 mL aliquot of the reaction mixture in a 6 mL glass test tube and centrifuge at 1500 rpm and 25±5°C for 5 minutes. Proceed to the next step if no precipitate is observed. If some precipitate is observed add 10 to 34 g citric acid to the mixture and continue heating for 10 to 30 min.
- Terminate the heating and cool the mixture to below 30°C. Measure the pH of the reaction mixture; it should be pH 0.8 to 1.5.
- Transfer equal amounts of the reaction mixture into four 500 mL centrifuge containers and balance the weight of each container using a top-loading balance.
- Centrifuge the reaction mixture at 1500 rpm and 25±5°C for 5 minutes. Transfer and pool all the ferric citrate supernatant to a clean 4 L Erlenmeyer flask.
- Repeat the above 2 steps for all of the ferric citrate reaction mixture.
- Place one-half of the ferric citrate supernatant in a 4 L Erlenmeyer flask and stir with a magnetic stir bar at high speed.
- Add slowly (over 20 min) 3.5 L of acetone (accurate volume acetone calculated as five fold the supernatant volume) and stir for an additional 10 min. A light-beige color precipitate forms.
- Transfer the suspension into four 500 mL centrifuge containers and balance the weight of each container using a top-loading balance.
- Centrifuge the ferric citrate suspension at 1500 rpm and 25±5°C for 5 minutes.
- Transfer and pool all the ferric citrate precipitate to a clean 4 L Erlenmeyer flask.
- Repeat the above 4 steps with the second half of the ferric citrate supernatant.
- Pool all ferric citrate precipitate, add 1.4 L of acetone and stir for 5 min.
- Transfer the suspension into four 500 mL centrifuge containers and balance the weight of each container using a top-loading balance.
- Centrifuge the suspension at 1500 rpm and 25±5°C for 5 minutes.
- Repeat the above 2 steps until all suspension is centrifuged.
- Transfer and pool all the ferric citrate precipitate to a clean 4 L Erlenmeyer flask.
- Repeat the above 5 steps two additional times (total of 3 washes).
- Label and weigh drying trays, and record their weight.
- Transfer the ferric citrate precipitate onto the drying dishes and dry at ambient temperature (25±5°C) for 16 hours.
- Place the drying trays with precipitate into a vacuum oven and dry at ambient temperature (25±5°C) and under vacuum (about 20 mm Hg) for 8 to 16 hours (until the material appears ready for grinding).
- Reduce the particle size of the ferric citrate in a porcelain mortar and pestle.
- Place the ferric citrate powder into a vacuum oven and dry at ambient temperature (25±5°C) and under vacuum (about 20 mm Hg) for 8 to 24 hours, until the material appears ready for sieving.
- Finely reduce the ferric citrate particle size in a porcelain mortar and pestle. Screen the ferric citrate powder through a 45 mesh size (355 micron) sieve.
- Transfer the ferric citrate powder into drying trays and place the trays in an oven to dry at 25±5°C and under high vacuum until the material appears dry (20 to 48 hours).
- Transfer the powder into pre-weighed plastic amber containers.
- Label and store the containers at ambient temperature and protected from light.

### Example 5. Method for Scalable Manufacture of Pharmaceutical-grade Ferric Citrate

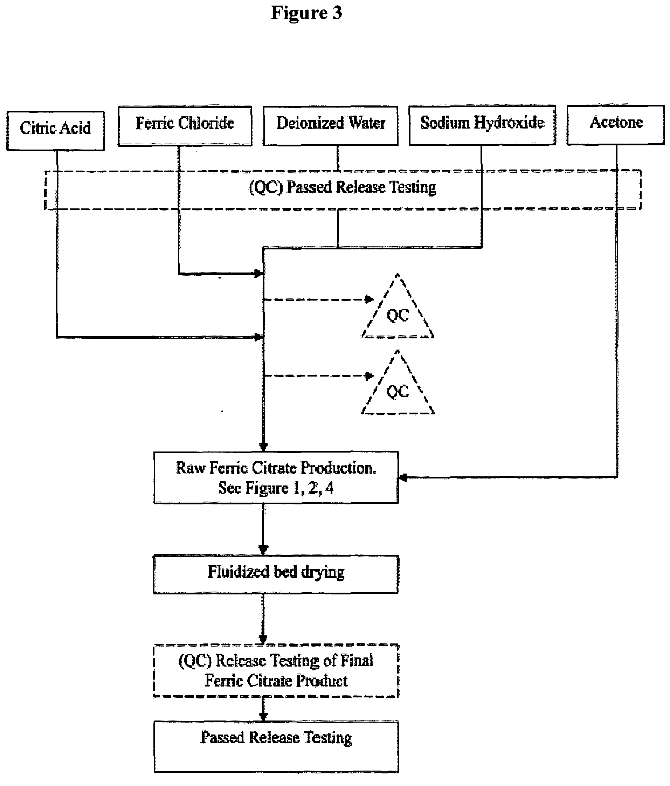

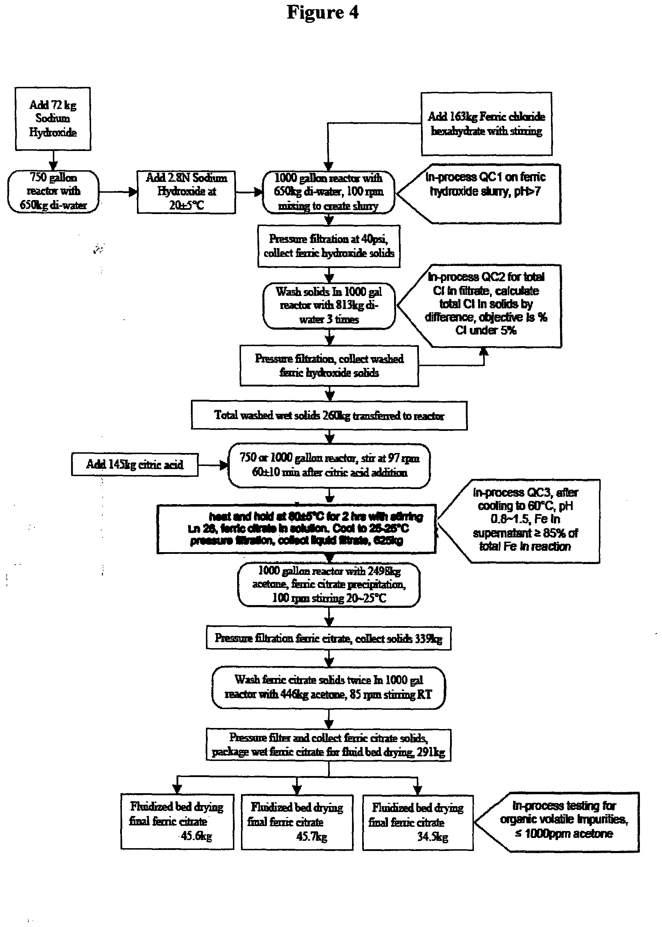

Described herein is a scalable manufacturing of pharmaceutical-grade ferric citrate. Preferably, the ferric citrate manufacturing process is capable of producing at least 125kg batches of pharmaceutical-grade ferric citrate. An overview of the ferric citrate manufacturing is shown in Figure 3. Details of the synthesis of ferric citrate are shown in Figure 4.

The scalable manufacturing process further employs fluidized bed dryer for drying wet ferric citrate and for attaining release specifications for organic volatile impurities. See Table A for the Manufacture Release Specifications for Pharmaceutical-Grade Ferric Citrate.

### Example 6. A Pharmaceutical-Grade Ferric Citrate

This invention provides a pharmaceutical-grade ferric citrate which complies with the Manufacture Release Specifications as shown below in Table A. The pharmaceutical-grade ferric citrate of consistent purity and quality can be prepared using the manufacturing process of the present invention. See figures 1-4 for the schematic diagram of the ferric citrate manufacturing and quality control process. The ferric citrate manufacturing and quality control process can be readily scaled to produce multi-kilogram batch sizes or scaled up to a manufacturing scale.

**Table A. Manufacture Release Specification for Pharmaceutical-Grade Ferric Citrate**

| **Test Item** | **Method** | **Limit of Specification** |
|---|---|---|
| Appearance | Visual | Light brown to beige powder |
| Purity of solid state ferric citrate | Calculate based on LC/MS - flow injection quantitation and profile, based on USP 25 <621>, <731>, <1086>, <736> | NLT 90 % w/w anhydrous basis |
| Assay content of ferric citrate non-related substances in solution state | LC/MS - flow injection profile, based on USP 25 <621>, <731>, <1086>, <736> | Run and report as % w/w anhydrous basis (attach table summary) |
| Assay content purity of ferric citrate and water adduct in solution state | LC/MS - flow injection quantitation, based on USP 25 <621>, <731>, <1086>, <736> | NLT 70 % w/w anhydrous basis |
| Assay content of citric acid related substance in solution state | LC/MS - flow injection quantitation, based on USP 25 <621>, <731>, <1086>, <736> | NLT 10 % w/w anhydrous basis |
| Assay content of other ferric citrate related substances in solution state (excluding citric acid) | LC/MS - flow injection profile, based on USP 25 <621>, <731>, <1086>, <736> | Run and report as % w/w anhydrous basis (attach table summary) |
| Assay content of ferric iron | Based on USP 25 ferric sulfate assay | NLT 16% w/w fresh weight basis |
| Limit of ferrous iron | Gravimetric method using potassium ferricynide, based on USP 25 <191> | NMT 1 % w/w fresh weight basis |
| Loss on drying | USP 25 <731> | NMT 20% |
| Hydrate (water content) | Karl Fischer Titration USP 25 <921> water determination, Method Ia (Direct Titration) | NMT 20% |
| Identification | Method A: based on USP 25 <191> ferric salt | Dark blue precipitate with K4Fe(CN)6 TS; reddish brown ppt with excess 1 N NaOH; deep red color not destroyed by mineral acids with NH4SCN TS |
| | | Sample solution gives light red color with pyridine and acetic anhydride |
| Limit of chloride | Based on USP 25 Ferric sulfate procedures | NMT 5% |
| Reside on ignition | Modified USP 25 <281> | Run and report |
| Organic Volatile Impurities | Based on USP 25 <467> | Acetone not more than 1000 ppm |
| Limit of acid insoluble substances | Gravimetric determination, based on USP 25, ferric sulfate | Not more than 0.02% w/w fresh weight basis |
| Trace / heavy metals | USP 25 method <231> or equivalent | As < 0.3 ppm |
| | | Others: run and report |
| | ICP for Zn, Cu, Sr, Ca, Na | |
| | GFAAS for As | |
| Lead, Cadmium | ICP-MS | Pb < 5 ppm Cd < 2 ppm |
| Mercury | Cold vapor / AA | Hg < 0.3 ppm |
| Total iron content | ICP | NLT 16% w/w fresh weight basis |
| Microbial / Mold and Yeast | USP Method <61> | |
| | Salmonella | Salmonella = negative |
| | E. Coli | E. Coli = negative |
| | Coliforms | Total Coliforms < 3 cfu/g |
| | Total aerobic count | Total aerobic count < 10 cfu/g |
| | Total combined mold and yeast | |
| | | Total mold and yeast < 20 cfu/g |

### Example 7. Raw Material Release Testing Specifications

**Table B. Ferric chloride hexahydrate Release Specifications**

| **Test Item** | **Method** | **Limit of Specification** |
|---|---|---|
| Appearance | Visual | Yellow to yellowish brown powder, crystals or chunks |
| Identification - Ferric salts | USP 25 <191> | Yield dark blue precipitate with potassium ferrocyanide TS |
| | | Form reddish brown precipitate with excess 1N NaOH |
| | | Red deep color with ammonium thiocyanate TS and color not destroyed by dilute mineral acids |
| Identification - Chloride | USP 25 <191> | Aqueous solution of ferric chloride yields with 0.1 N silver nitrate TS a white, cruddy precipitate, which is insoluble in nitric acid but is soluble in a slight excess of 6 N ammonium hydroxide |
| Heavy metals | ICP-MS | As < 0.1 ppm |
| | | Cd < 0.1 ppm |
| | | Hg < 0.1 ppm |
| | | Pb < 0.1 ppm |
| Assay - Ferric iron | USP 25, Assay ferric sulfate, p. 2303 | >18% |

**Table C. Sodium Hydroxide Release Specifications**

| **Test Item** | **Method** | **Limit of Specification** |
|---|---|---|
| Appearance | Visual | White pellets, odorless |
| Identification - Sodium | USP25, <191> | No precipitate is formed with potassium carbonate |
| | | A dense precipitate is formed with potassium pyroantimonate |
| | | An intense yellow color to a non-luminous flame |

**Table D. Deionized Water Release Specification**

| **Test Item** | **Method** | **Limit of Specification** |
|---|---|---|
| Appearance | Visual | Clear, colorless and odorless |
| Mineral scan | ICP-MS | As < 0.001 ml/L |
| | | Cd < 0.0002 ml/L |
| | | Pb < 0.001 ml/L 0.02 |
| | | Hg < µg/L |
| Total Organic Carbon | Standard Method for the Examination of Water and Wastewater, 20^{th} ed. | < 1 mg/L |
| Total Hardness | Standard Method for the Examination of Water and Wastewater, 20^{th} ed. | < 4 mg/L |
| Total Plate Count | USP 25 method <61> | <10 cfu |
| Total Coliform Count | USP 25 method <61> | <3 cfu |

**Table E. Acetone Release Specification**

| **Test Item** | **Method** | **Limit of Specification** |
|---|---|---|
| Appearance | Visual | Clear colorless liquid |
| Identification - Acetone | USP 25, p.2502, FTIR | The IR absorption of a thin film between KBr plates exhibits a strong band at about 5.8 µm; a strong region of absorption between 6.8 and 7.5 µm, with maxima at about 7.0 and 7.3 µm; a strong maximum at about 8.2 µm; and a weak maxima at about 9.2 and 11.0 µm. |
| Assay | From manufacturer's Certificate of Analysis (result from GC method preferred) | NLT 99.5% |
| Aldehyde (as HCHO) | From manufacturer's Certificate of Analysis | NMT 0.002% |
| Isopropyl alcohol | From manufacturer's Certificate of Analysis | NMT 0.05% |
| Methanol | From manufacturer's Certificate of Analysis | NMT 0.05% |
| Residue after evaporation | From manufacturer's Certificate of Analysis | NMT 5 ppm |
| Acids | From manufacturer's Certificate of Analysis (result from titrimetric test) | NMT 0.0003 meq |
| Bases | From manufacturer's Certificate of Analysis (result from titrimetric test) | NMT 0.0006 meq |
| Water | From manufacturer's Certificate of Analysis | NMT 2% |
| Insoluble substances | From manufacturer's Certificate of Analysis or filtered through ≤0.45 µm filter | filtered through ≤0.45 µm filter |

**Table F. Citric Acid Release Specification**

| **Test Item** | **Method** | **Limit of Specification** |
|---|---|---|
| Appearance | Visual | White or colorless crystals or powder |
| Identification - Citrate | USP 25 <191> | A light red color is produced |

### Example 8. Final Product Manufacture Release Testing Methods

The following tests are performed to ensure the final ferric citrate product prepared according to the method or process described herein complies with the established Manufacture Release Specification as shown in Table A. The Manufacture Release Specification may be readily modified or revised by one of ordinary skill in the art following the teaching of this invention to enhance the purity and safety of the pharmaceutical-grade ferric citrate for human use.
(a) Based on USP 25 <191> Gravimetric method using potassium ferricyanide, p. 1918 - Limit of Ferrous Iron in Ferric Citrate
(b) Based on USP 25, ferric sulfate assay, p. 728 - Limit of Chloride in Ferric Citrate
(c) Based on USP 25, ferric sulfate assay, p. 728 - Limit of Acid Insoluble Substances in Ferric Citrate
(d) Based on USP 25, ferric sulfate assay, p. 728 - Assay Content of Ferric Iron in Ferric Citrate
(e) Based on USP 25 <467> - Determination of Acetone in Ferric Citrate Samples by GClFID Headspace
(f) Based on USP 25 <191> Ferric Salts; Citrate, p. 1918 - Identification of Ferric Citrate
(g) Based on USP 25 <621>, p. 1988-1995, <731>, <1086>, p. 2157-2159, <736>, p. 2029-2033 - LCMS Flow-Injection Quantitation and Profile of Ferric Citrate and Related Substances
(h) USP 25 <731> Loss on Drying
(i) USP 25 <921> Water Determination, Method Ia (Direct Titration)
(j) ICP for Zn, Cu, Sr, Ca, Na and total iron
(k) GFAAS for As
(l) ICP-MS for lead and cadmium
(m) Cold vapor/AA for mercury
(n) Residue on ignition
(o) Microbial / mold and yeast

### Example 9 Methods of Using and Testing the Pharmaceutical-Grade Ferric Citrate in Patients In Vivo

### A. Handling and Forms of Test Compositions

Ferric citrate is supplied in 500 mg capsules, whereas the placebo will be provided in identical-looking capsules (indistinguishable from those containing the active drug); the placebo capsules will contain sorbitol and colorant to match the powder color of the active capsules. The placebo capsule shells will be identical to the active capsule shells.

### Storage

All study drug supplies must be stored under secure conditions and are not to be used after their expiration date, which is imprinted on the study drug container. The study drugs should be kept under controlled conditions (15 to 30°C; 59 to 86°F) in a tightly closed container, protected from light.

### Dosage

A recent pilot study compared ferric citrate (3 g daily) to calcium carbonate (3g daily) for reducing serum P0₄ in patients with End Stage Renal Disease (ESRD). Although ferric citrate caused a significant decrease in serum P04, it was not as effective as calcium carbonate. This dose of ferric citrate was associated with mild, but tolerable GI symptoms.

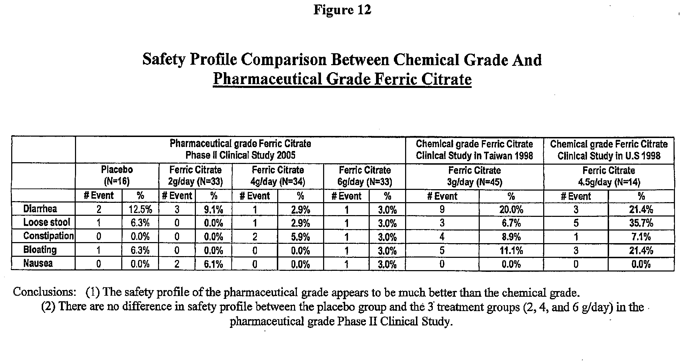

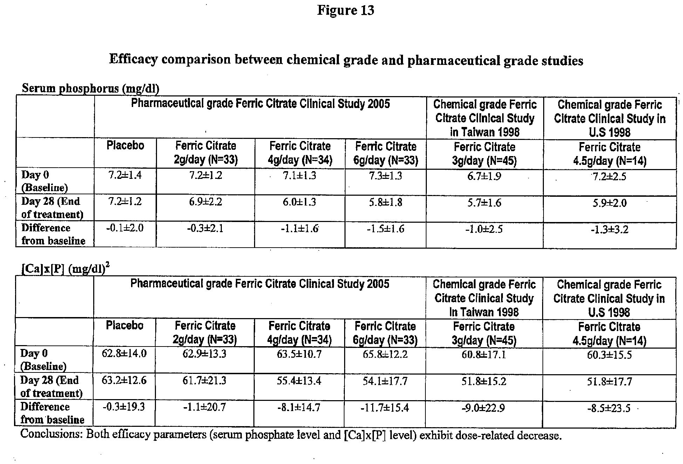

As shown in Figures 12 and 13, treatments using pharmaceutical-grade ferric citrate provide several advantages over chemical grade ferric citrate. In general, while pharmaceutical-grade ferric citrate demonstrates an efficacy approximately equal to that of chemical grade ferric citrate, it achieves this result with less adverse side effects than chemical grade ferric citrate.

Figure 12 also indicates that adverse side effects associated with administering pharmaceutical-grade ferric citrate were not statistically different from those associated with the placebo. An advantage of this safety profile is that an individual patient may have his dosing of pharmaceutical-grade ferric citrate titrated over a broad range of doses with less concern about side effect. In this way, a patient's individual treatment may be tailored to suit his specific needs and tolerances.

The invention relates to a dose of 6 grams of ferric citrate per day. Also disclosed is that doses of ferric citrate chosen for study or treatment may be from 1 to 30 grams of ferric citrate per day. In part, this may depend on the nature of the formulation provided. For example, ferric citrate capsules may be administered up to a daily dose of about 15 grams/day, whereas the tablet form may be administered up to 30 grams/day.

### Titration of Optimal Dosage for a Subject

In the context of this invention, the term "subject" refers to either a human or non-human animal.

The optimal dosage of an individual subject or groups may be determined as follows. A dose of approximately one or two grams per day is merely suggested as an illustrative starting dose. The daily dose may be increased as needed until the desired result is observed. The invention relates to a dose of 6 grams of ferric citrate per day.

### Illustrative Example of a Dosage Regimen

An example of a dosing regimen is provided below. This is not meant to limit the invention as to how an effective amount of ferric citrate is selected, or the form in which it is provided or the frequency of administering the composition per day. The following merely illustrates how ferric citrate and placebo may be administered; e.g., as 500 mg capsules of identical appearance. All patients may receive (in a blinded fashion) 4 capsules with each of three meals, on a daily basis, for 28 days. Patients will be instructed to take the study medication within 10 minutes of finishing their meals (breakfast, lunch, and dinner).

The number of placebo, and active capsules to be taken at each meal, are as follows:

### Placebo arm of the study

4 placebo capsules, with breakfast
4 placebo capsules, with lunch
4 placebo capsules with dinner

### Ferric citrate 2 g per day arm of the study

1 ferric citrate capsule and 3 placebo capsules with breakfast
1 ferric citrate capsule and 3 placebo capsules with lunch
2 ferric citrate capsules and 2 placebo capsules with dinner

### Ferric citrate 4 g per day arm

2 ferric citrate capsules and 2 placebo capsules with breakfast
3 ferric citrate capsule and 1 placebo capsule with lunch 3 ferric citrate capsules and 1 placebo capsule with dinner

### Ferric citrate 6 g per day arm

4 ferric citrate capsules with breakfast
4 ferric citrate capsules with lunch
4 ferric citrate capsules with dinner

### B. Clinical Schedule and Assessments

### Duration of Study Treatment

Each patient's participation in the trial lasts for up to 8 weeks: the screening period (approximately 1-2 weeks), a 1-2 week washout, and 4 weeks of treatment with study medication.

### Screening Visit 1 (Study Days -30 to -15)

The following procedures will be performed at the first screening visit:
1. Medical history, including concomitant medications.
2. Demographic data.
3. Physical examination, including height, weight, and vital signs.
4. Dietary interview, using 24 hour recall method, to assess dietary intake of calcium and phosphorous, three times during screening period, to include one dialysis day, one non-dialysis day, and one weekend day. Note: Dietary interview may be also performed, in part or in whole, during the washout period.
5. Laboratory assessment:
   - Hematology: complete blood count (CBC) with differential, platelets.
   - Chemistries: sodium, potassium, chloride, bicarbonate, blood urea nitrogen (BUN), creatinine, glucose (random), aspartate transaminase (AST), alanine transaminase (ALT), alkaline phosphatase (ALP), total bilirubin, total protein, albumin, serum calcium, serum phosphate, magnesium
   - Total and LDL cholesterol levels
   - Serum (3-HCG for women of childbearing potential
   - Iron panel: serum iron, ferritin, transferrin saturation percentage, and total iron binding capacity.
6. 12-lead ECG.
7. Patients will be given instructions for the Washout Period (Study Days - 14 to -1):
   a. All phosphate-binding agents will be discontinued at Day - 14
   b. Any iron therapy (oral or intravenous) will be discontinued at Day - 14
   c. Patients who have been receiving a stable dose of vitamin D or calcitriol for I month prior to enrollment will be instructed to maintain their current dose throughout the study
   d. Patients will be advised to avoid changes in diet, calcium or magnesium containing antacids (other medications).

### Screening Visit 2 (Study Days -7 +/- 1 day)

### 1. Laboratory Assessment:

### Serum P0₄

Note: Patients with a Day - 7 serum P0₄ ≥ 5.5 mg/dL and ≤10 mg/dL may be randomized before the 2-week washout is complete. The day of randomization will automatically become Day 0.

Note: Patients with a Day - 7 phosphate level of ≥ 10 mg/dL will be removed from the study and instructed to resume their pre-study medications.

### Study Day 0 (Randomization And Dosing)

1. Physical examination, including weight and vital signs.
2. Adverse event query.
3. Concomitant medication query.
4. Baseline Laboratory assessments:
   - Serum P04
   - Serum Ca
   - Iron panel: serum iron, ferritin, transferrin saturation percentage, and total iron binding capacity
   Note: The Baseline Laboratory Assessments may be done up to 3 days prior to Day 0
5. Patients with a P0₄ level ≥ .5 mg/dL and ≤ 10 mg/dL will be randomized and a 15-day supply of study medication will be dispensed.

Note: Patients should be instructed to begin taking study medication within 10 minutes of completing their next meal on Day 0.

### Study Day 14 (Midpoint Evaluation)

The following procedures will be performed at Study Day 14 +/- 1 day.
1. Physical examination including weight and vital signs.
2. Adverse event query.
3. Concomitant medication query.
4. Dispense an additional 15-day supply of study medication. All returned capsules should be counted and recorded in the Case Report Form.
5. Laboratory assessment:
   - Hematology: CBC with differential, platelets.
   - Chemistries: sodium, potassium, chloride, bicarbonate, BUN, creatinine, glucose (random), AST, ALT, ALP, total bilirubin, total protein, albumin, calcium, phosphate, magnesium.
   - Iron panel: serum iron, ferritin, transferrin saturation percentage, and total iron binding capacity.
   - Total and LDL cholesterol levels.

Note: Patients with a Day 14 phosphate level of > 10 mg/dL will be removed from the study and instructed to resume their pre-study medications.

### Study Day 28 (End Of Study Evaluation)

The following procedures will be performed at Study Day 28 +/- 1 day or on the day of early termination.
1. Physical examination including weight and vital signs
2. Adverse event query.
3. Concomitant medication query.
4. Laboratory assessment:
   a. Hematology: CBC with differential, platelets
   b. Chemistries: sodium, potassium, chloride, bicarbonate, BUN, creatinine, glucose (random), AST, ALT, ALP, total bilirubin, total protein, albumin, calcium, phosphate, magnesium.
   c. Total and LDL cholesterol levels
   d. Iron panel: serum iron, ferritin, transferrin saturation percentage, and total iron binding capacity.
5. 12-lead ECG
6. Patients will be instructed to resume their pre-study medications after completing the study.

### C. Data Management and Analysis

GloboMax will be the primary data management, monitoring, and coordinating center. Case report forms (CRF) will be provided for each subject. Subjects will not be identified by name or initials on CRFs. The CRF will be monitored at the clinical sites and collected by GloboMax's study monitor. Audited CRFs will be used to create electronic data files.

Three major categories of endpoints reflect biochemical and clinical issues being addressed at the outset. Additional clinical and biochemical issues are addressed as they arise. Therefore, the endpoints are not meant to limit the totality of relevant findings and measurements collected in these, or future studies.

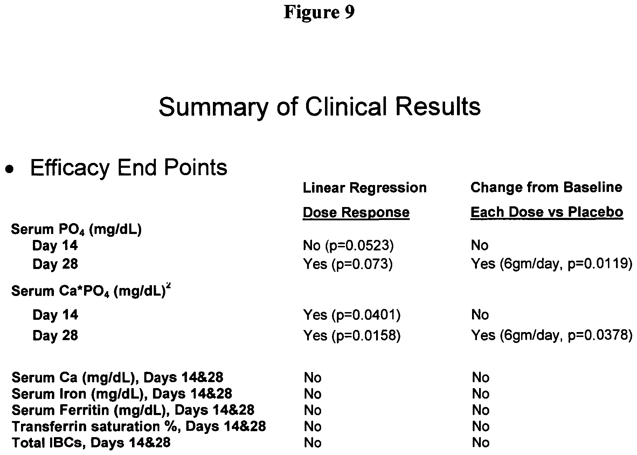

### Primary Endpoints (see Figures 9-10)

- The change in serum P0₄ concentration at Days 14 and 28 from baseline.

### Secondary Endpoints (see Figures 9-10)

- The change in serum calcium concentration at Days 14 and 28 from baseline.
- The change in iron, ferritin, transferrin saturation percentage, and total iron binding capacity at Days 14 and 28 from baseline.
- The change in the Ca'PO₄ product at Days 14 and 28 from baseline.

It is further noted that in comparison to chemical grade ferric citrate, the pharmaceutical grade ferric citrate demonstrates similar efficacy. See Figure 13. However, the safety profiles indicate that the pharmaceutical grade generally results in less adverse clinical effects. See Figure 13.

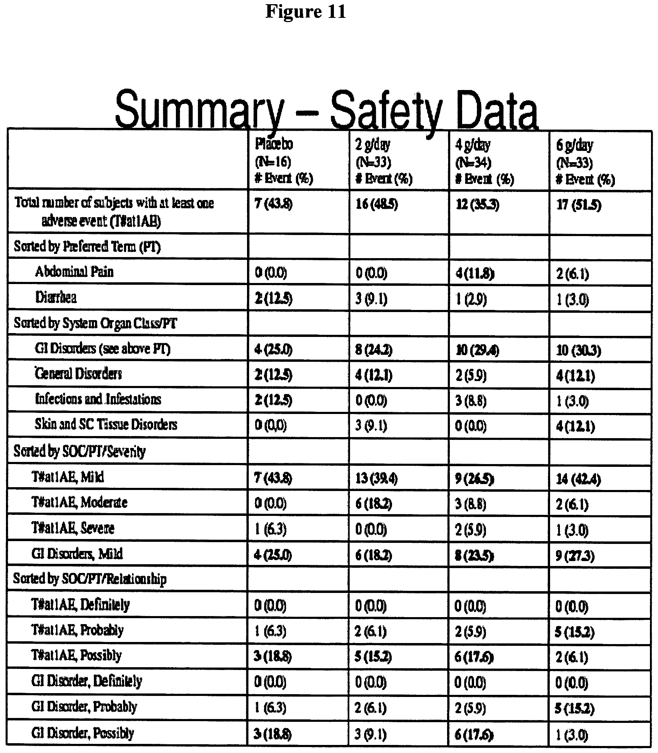

### Safety Endpoints (see Figure 11)

Safety will be monitored by recording adverse events (Figure 11) and the results of physical examinations, vital signs and laboratory tests at each study visit.

Specific rules for withdrawal from the study, based on laboratory data, have also been set up to ensure patient safety. A nonlimiting example of such criteria is illustrated by the following:
If a patient's serum phosphate level increases to ≥10 mg/dL at any time during the study period, the patient will be withdrawn from the study.

Specific studies have also shown that pharmaceutical grade ferric citrate possesses similar efficacy to chemical grade ferric citrate. (See Figure 13). However, the pharmaceutical grade generally affords a significantly more desirable safety profile as shown in Figure 12. This indicates an important advance in regulating serum phosphate levels.

## Claims

1. A pharmaceutical composition for use in treating hyperphosphatemia, the composition comprising a form of pharmaceutical grade ferric citrate and a pharmaceutically suitable carrier, wherein the amount of pharmaceutical grade ferric citrate administered per day is 6 grams per day, and wherein the pharmaceutical grade ferric citrate is administered in a tablet or capsule form, wherein the pharmaceutical grade ferric citrate is obtainable by a method comprising the steps of:
(a) dissolving an appropriate amount of ferric chloride hexahydrate in water to form a ferric chloride hexahydrate solution;
(b) dissolving an appropriate amount of NaOH in water to form a NaOH solution;
(c) mixing the ferric chloride hexahydrate solution and NaOH solution to form a solution with Fe(OH)₃ precipitate;
(d) maintaining the pH of the solution with Fe(OH)₃ precipitate above 7.0;
(e) isolating the Fe(OH)₃ precipitate;
(f) washing the Fe(OH)₃ precipitate three times with water;
(g) suspending the Fe(OH)₃ precipitate in water;
(h) adding citric acid to the Fe(OH)₃ precipitate to form a ferric-organic acid solution;
(i) stirring and heating the ferric-organic acid solution at 90-100°C for 30 to 120 minutes;
(j) removing solids in the ferric-organic acid solution by adding citric acid;
(k) allowing the ferric-organic acid solution to cool to below 30°C;
(l) maintaining the pH of the ferric-organic acid solution to between 0.8-1.5;
(m) filtering the ferric-organic acid solution to obtain a liquid filtrate;
(n) mixing acetone and liquid filtrate to form ferric citrate;
(o) isolating ferric citrate;
(p) washing ferric citrate with acetone three times; and
(q) drying the ferric citrate.

2. The pharmaceutical composition for use of claim 1 in an orally administrable form.

3. The pharmaceutical composition for use of claim 2, wherein the orally administrable form is a tablet or capsule.

## Patentansprüche

1. Pharmazeutische Zusammensetzung zur Verwendung bei der Behandlung von Hyperphosphatämie, wobei die Zusammensetzung eine Form von Eisen(III)-citrat pharmazeutischer Qualität und einen pharmazeutisch geeigneten Träger umfasst, wobei die verabreichte Menge an Eisen(III)-citrat pharmazeutischer Qualität 6 Gramm pro Tag beträgt und wobei das Eisen(III)-citrat pharmazeutischer Qualität in einer Tabletten- oder Kapselform verabreicht wird, wobei das Eisen(III)-citrat pharmazeutischer Qualität durch ein Verfahren erhältlich ist, das die folgenden Schritte umfasst:
(a) Lösen einer geeigneten Menge von Eisen(III)-chloridhexahydrat in Wasser zur Bildung einer Eisen(III)-chloridhexahydrat-Lösung;
(b) Lösen einer geeigneten Menge von NaOH in Wasser zur Bildung einer NaOH-Lösung;
(c) Mischen der Eisen(III)chloridhexahydrat-Lösung und der NaOH-Lösung zur Bildung einer Lösung mit Fe(OH)₃-Niederschlag;
(d) Halten des pH-Werts der Lösung mit Fe(OH)₃-Niederschlag über 7,0;
(e) Isolieren des Fe(OH)₃-Niederschlags;
(f) dreimaliges Waschen des Fe(OH)₃-Niederschlags mit Wasser;
(g) Suspendieren des Fe(OH)₃-Niederschlags in Wasser;
(h) Zugeben von Citronensäure zu dem Fe(OH)₃-Niederschlag zur Bildung einer eisen(III)-organischen sauren Lösung;
(i) Rühren und Erhitzen der eisen(III)-organischen sauren Lösung auf 90-100 °C für 30 bis 120 Minuten;
(j) Entfernen von Feststoffen in der eisen(III)-organischen sauren Lösung durch Zugabe von Citronensäure;
(k) Abkühlenlassen der eisen(III)-organischen sauren Lösung auf unter 30°C;
(l) Halten des pH-Werts der eisen(III)-organischen sauren Lösung zwischen 0,8-1,5;
(m) Filtrieren der eisen(III)-organischen sauren Lösung zum Erhalt eines flüssigen Filtrats;
(n) Mischen von Aceton und flüssigem Filtrat zur Bildung von Eisen(III)-citrat;
(o) Isolieren von Eisen(III)-citrat;
(p) dreimaliges Waschen von Eisen(III)-citrat mit Aceton; und
(q) Trocknen des Eisen(III)-citrats.

2. Pharmazeutische Zusammensetzung zur Verwendung nach Anspruch 1 in einer oral verabreichbaren Form.

3. Pharmazeutische Zusammensetzung zur Verwendung nach Anspruch 2, wobei die oral verabreichbare Form eine Tablette oder Kapsel ist.

## Revendications

1. Composition pharmaceutique pour une utilisation dans le traitement de l'hyperphosphatémie, la composition comprenant une forme de citrate ferrique de qualité pharmaceutique et un support pharmaceutiquement approprié, dans laquelle la quantité de citrate ferrique de qualité pharmaceutique administrée par jour est de 6 grammes par jour, et dans laquelle le citrate ferrique de qualité pharmaceutique est administré sous une forme de comprimé ou de capsule, dans laquelle le citrate ferrique de qualité pharmaceutique peut être obtenu par un procédé comprenant les étapes consistant à :
(a) dissoudre une quantité appropriée de chlorure ferrique hexahydraté dans l'eau pour former une solution de chlorure ferrique hexahydraté ;
(b) dissoudre une quantité appropriée de NaOH dans l'eau pour former une solution de NaOH ;
(c) mélanger la solution de chlorure ferrique hexahydraté et la solution de NaOH pour former une solution avec un précipité de Fe(OH)₃ ;
(d) maintenir le pH de la solution avec le précipité de Fe(OH)₃ au-dessus de 7,0 ;
(e) isoler le précipité de Fe(OH)₃ ;
(f) laver le précipité de Fe(OH)₃ trois fois avec de l'eau ;
(g) mettre en suspension le précipité de Fe(OH)₃ dans l'eau ;
(h) ajouter de l'acide citrique au précipité de Fe(OH)₃ pour former une solution d'acide ferrique-organique ;
(i) agiter et chauffer la solution d'acide ferrique-organique à 90-100 °C pendant 30 à 120 minutes ;
(j) éliminer des solides dans la solution d'acide ferrique-organique par ajout d'acide citrique ;
(k) laisser refroidir la solution d'acide ferrique-organique jusqu'à une température inférieure à 30 °C ;
(l) maintenir le pH de la solution d'acide ferrique-organique entre 0,8 et 1,5 ;
(m) filtrer la solution d'acide ferrique-organique pour obtenir un filtrat liquide ;
(n) mélanger de l'acétone et le filtrat liquide pour former du citrate ferrique ;
(o) isoler le citrate ferrique ;
(p) laver le citrate ferrique trois fois avec de l'acétone ; et
(q) sécher le citrate ferrique.

2. Composition pharmaceutique pour une utilisation selon la revendication 1 sous une forme administrable par voie orale.

3. Composition pharmaceutique pour une utilisation selon la revendication 2, dans laquelle la forme administrable par voie orale est un comprimé ou une capsule.
